# EUROPEAN PATENT APPLICATION

(11) **EP 3 819 308 A1**
(43) Date of publication of application: **12.05.2021**
(21) Application number: 19207738.6
(22) Date of filing: 07.11.2019
(51) Int. Cl.: C07K 14/605

(54) **PROCESS FOR THE MANUFACTURE OF DERIVATIZED AMINO ACIDS**

(71) Applicant: Fresenius Kabi iPSUM S.r.l., 20060 Cassina de' Pecchi - Milano (IT)
(72) Inventor: RICCI, Antonio, 45010 Villadose (IT); CALLENS, Roland, 45010 Villadose (IT); NUIJENS, Timo, 45010 Villadose (IT); CABRI, Walter, 45010 Villadose (IT)
(74) Representative: Fresenius Kabi Deutschland GmbH

(57) **Abstract**

The present invention relates to peptide synthesis, and in particular to a process for the manufacture of protected amino acids substituted at their side-chains. In addition, the present invention relates to the use of such protected amino acids in the preparation of GLP-1 analogues, such as for instance liraglutide and semaglutide.

## Description

### Field of the invention

The present invention relates to amino acids and peptide synthesis. In particular, it relates to a process for the manufacture of derivatized and protected amino acids substituted at their side-chains. More in particular, the present invention relates to a process for the manufacture of protected amino acids and their use in the preparation of GLP-1 analogues, such as for instance liraglutide and semaglutide.

### Background of the invention

Glucagon-like peptide-1 (GLP-1) is a naturally occurring peptide hormone with one of the most potent insulinotropic activities. GLP-1 is part of a longer peptide produced and processed to GLP-1 in the pancreas and the intestine. Glucagon-like peptide 1 (GLP-1) receptor agonists, or GLP-1 analogues, are a relatively new group of injectable drugs for the treatment of type 2 diabetes. One of their advantages over older insulin secretagogues, such as sulfonylureas or meglitinides, is that they have a lower risk of causing hypoglycemia.

Among GLP-1 analogues, liraglutide and semaglutide are highly similar peptides of a length of 30 amino acids, which just differ in the amino acid in position 2 (aa², which is alanine, Ala, in liraglutide and α-aminoisobutyric acid, Aib, in semaglutide) and in the side chain attached to the lysine in position 20 (Lys²⁰), i.e. the lipophilic albumin binding moiety. Attached to the ε-amino group of such lysine, liraglutide has a Glu-spaced palmitic acid (indicated as Pal-Glu), while semaglutide bears an octadecandioic-acylated-glutamic acid plus a further spacer.

Liraglutide and semaglutide are represented by using the "three letter code" for amino acids/peptides, according to the following: 
1                    5                    10                    15                    20
His-X₁-Glu-Gly-Thr-Phe-Thr-Ser-Asp-Val-Ser-Ser-Tyr-Leu-Glu-Gly-Gln-Ala-Ala-Lys(X₂)-
          25                    30   31
Glu-Phe-Ile-Ala-Trp-Leu-Val-Arg-Gly-Arg-Gly
 wherein the numbering of aminoacidic residues starts with 1, indicating N-terminal histidine, and finishes with 31, indicating C-terminal glycine,
and wherein

in liraglutide
X₁ is Ala and
X₂ is N-(1-oxohexadecyl)-L-γ-glutamyl (also indicated as Pal-Glu), depicted herebelow and

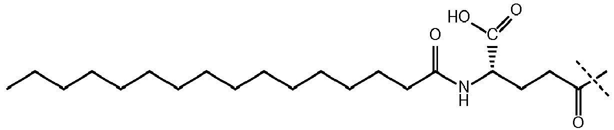

in semaglutide
X₁ is Aib (α-aminoisobutyric acid) and
X₂ is N-(17-carboxy-1-oxoheptadecyl)-L-γ-glutamyl-2-[2-(2-aminoethoxy)ethoxy] acetyl-2-[2-(2-aminoethoxy)ethoxy]acetyl (also referred to as [HOOC-(CH₂)₁₆-CO-yGlu-OH]-AEEA-AEEA) depicted herebelow 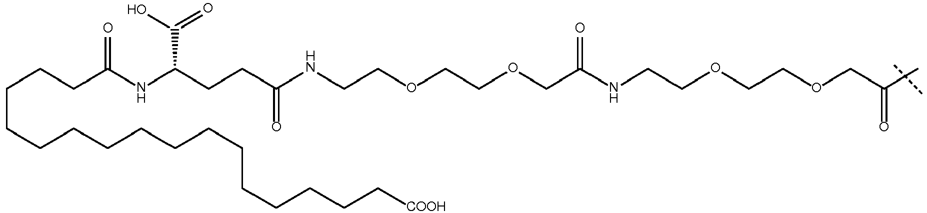 and wherein X₂ is attached to the ε-amino group of Lys²⁰ through amide bond where indicated by

The preparation of GLP-1 analogues has been described in several patent applications. Both sequential SPPS (Solid Phase Peptide Synthesis) and fragment-based approaches were pursued.

In addition, processes were described wherein either the bulky side-chains are connected to the peptide backbone during peptide elongation or they are attached only onto the final peptide.

For instance, EP1863839 discloses a synthesis of semaglutide wherein the Lys²⁰ side-chain is attached as a whole in one step in solution phase onto the full-length peptide. EP2757107 discloses a similar approach for the preparation of liraglutide, wherein the Lys²⁰ side-chain is attached as a whole in one step in solid phase.

WO2019120639 discloses a preparation of semaglutide wherein Lys²⁰ is attached to the growing peptide chain on solid phase and its side-chain is then built up before the further stepwise elongation of the peptide.

CN104356224 discloses a stepwise SPPS preparation of semaglutide wherein a fully derivatized and protected Lys²⁰ is used, which is synthesized through couplings of Boc-protected building blocks in standard conditions.

CN106749613 discloses a preparation of a fully derivatized and protected Lys²⁰, which is synthesized through couplings of Fmoc-protected building blocks in standard conditions.

The need to develop an efficient, simple and industrially viable process for the preparation of protected amino acids substituted at their side-chains is however still actual.

The present invention provides a novel method for the manufacturing of protected amino acids substituted at their side-chains, wherein a silyl moiety formed *in situ* is used as a temporary protective group during the coupling of building blocks. The present method improves the productivity and reduces the excess of starting material required with respect to the processes currently present in the prior art. Such new process can therefore be applied to the manufacturing of building blocks for the preparation of GLP-1 analogues, like liraglutide and semaglutide. In particular, the present invention relates to protected lysine substituted at its side-chain, that is used in position 20 of a GLP-1 analogue.

### Summary of the invention

The present invention provides a process for the preparation of a compound of formula I or a salt thereof, 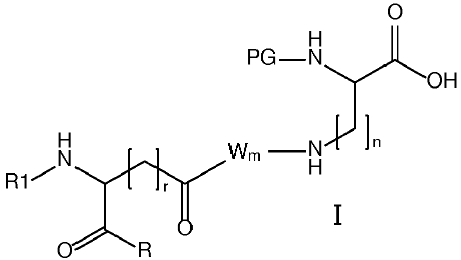 wherein
PG is an amino protective group selected from the group consisting of Fmoc, Boc, Alloc, benzoylcarbonyl and their derivatives,
n is an integer from 1 to 10;
m is 0 or 2;
r is an integer from 0 to 10;
each W is independently a group of formula XI: 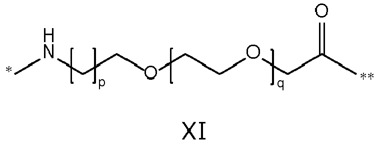 wherein
   p is an integer from 1 to 3;
   q is an integer from 1 to 2;
      * denotes the point of attachment to the carbonyl in formula I or to the C-terminal end of the further group W;
      ** denotes the point of attachment to NH in formula I or to the N-terminal end of the further group W;
R is OR2 or NR3R4, wherein R2, R3 and R4 are selected from the group consisting of hydrogen, linear or branched C₁-C₆ alkyl, aryl and arylalkyl;
R1 is selected from a group of formula XII and a group of formula XIII: 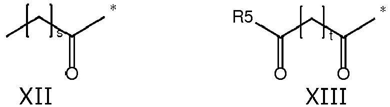 wherein
   * denotes the point of attachment to NH;
   R5 is OR2 or NR3R4, as defined above;
   s is an integer from 0 to 20
   t is an integer from 1 to 20.

The process according to the present invention comprises: step a) reacting a compound of formula X: 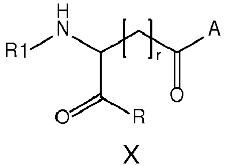 wherein A is hydroxy or an activating group, R1 and R are as defined above, but,
when R is OR2, R2 is not hydrogen; with a persilylated compound of formula IX: 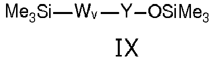 wherein
v is 0, 1 or 2, and Y is null or a group of formula VIII: 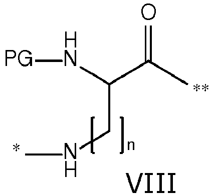 wherein
* denotes the point of attachment to Wᵥ or to SiMe₃;
** denotes the point of attachment to OSiMe₃, and n is as defined above;
and which is selected from: 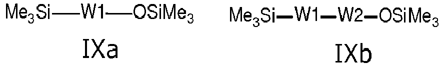 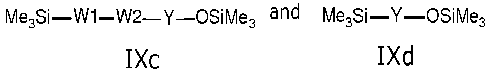 wherein Y is not null and wherein W1 and W2 are the same or different W groups, as defined above;
such reaction resulting in a compound of formula Iv: 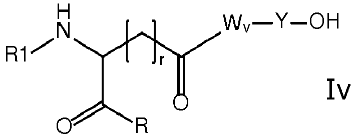 which, depending on the compound of formula IX used, is a compound of formula: 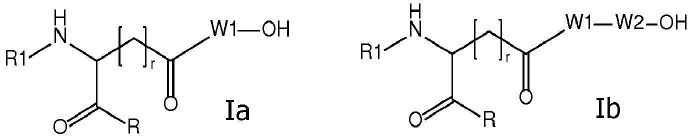 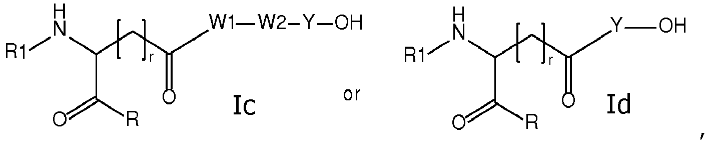 respectively.

A compound of formula Ic or Id corresponds to a compound of formula I.

A compound of formula Ia or Ib is optionally converted to a compound of formula I (step b).

A compound of formula Ia, optionally activated at its carboxylic acid with an activating group A, is reacted with a persilylated compound of formula IXe 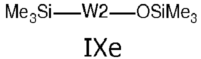 resulting in a compound of formula Ib (step c).

A compound of formula Ib, optionally activated at its carboxylic acid with an activating group A, is reacted with a compound of formula IXd, resulting in a compound of formula Ic (step d).

In a preferred embodiment, the process of present invention relates to the preparation of a compound of formula I wherein n is 4 and r is 2.

In particular, in a preferred embodiment when n is 4, r is 2, R is OR2 wherein R2 is tert-butyl, the process provides a compound of formula Ie, with the following structure: 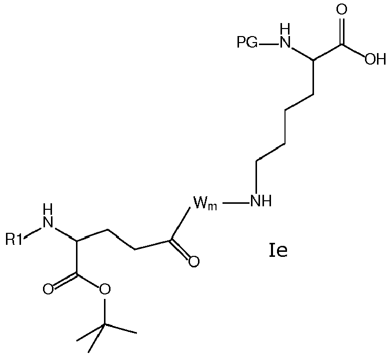 which is a protected side-chain derivatized lysine.

In a more preferred embodiment, when n is 4, r is 2, m is 0, R is OR2 wherein R2 is tert-butyl, PG is Fmoc and R1 is 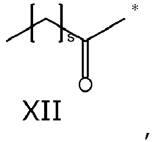 wherein s is 14, then the process provides a compound **2,** which is a compound of formula Id and a lysine derivative with the following structure:

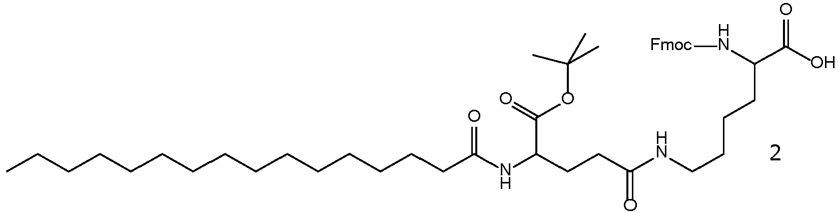

In another more preferred embodiment, when n is 4, r is 2, m is 2, R is OR2 wherein R2 is tert-butyl, PG is Fmoc and R1 is 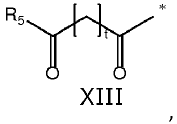 wherein t is 16 and R5 is OR2 wherein R2 is tert-butyl, W1 and W2 are the same group of formula XI wherein p and q are 1, then the process provides a compound **1,** which is a compound of formula Ic and a lysine derivative with the following structure:

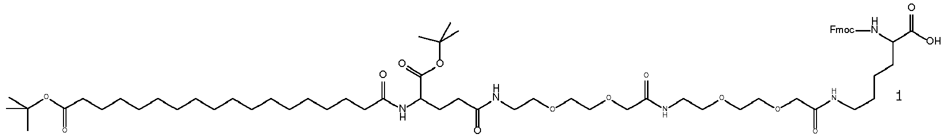

### Detailed description of the invention

The new synthetic approach developed by the inventors for the preparation of a compound of formula I exploits the use of silyl groups as temporary protective groups that can be inserted and removed *in situ* in mild conditions without isolation of the intermediate protected compounds.

The advantages of this approach are:
1) unprotected (partly or fully) starting materials are used, which are cheaper and easier to produce than their protected counterparts;
2) silylation and desilylation steps of the intermediate compounds are performed under mild reaction conditions, whereas the protection/deprotection steps reported in the prior art (mainly Boc and Fmoc protective groups) involve the use of harsh reaction conditions, which may compromise the quality of such intermediate compounds and, consequently, of the final product;
3) since unprotected (partly or fully) starting materials are more soluble than the corresponding Boc or Fmoc protected counterparts, a higher versatility of the preparation reactions is allowed, for instance in the choice of solvents;
4) a lower amount of equivalents of starting materials is used than in the solid phase approaches, with a considerable saving in cost and productivity.

The silylation reaction is performed with a silylating reagent, which is selected from the group comprising N-methyl-N-trimethylsilylacetamide (TMA), trimethylsilyl cyanide (TMSCN), bis(trimethylsilyl)acetamide (BSA), 1,3-bis(trimethylsilyl)urea (BSU), trimethylchlorosilane (TMCS), and trimethyliodosilane (TMIS). Preferably, the silylation reaction is performed with N-methyl-N-trimethylsilylacetamide.

Such silylation reaction is performed in a suitable solvent, which is a non protic organic solvent, selected in the group comprising nitriles (e.g. acetonitrile), ethers (e.g. tetrahydrofuran or dioxane), hydrocarbons (e.g. toluene), esters (e.g. ethyl or isopropyl acetate), chlorinated solvents (e.g. dichloromethane), or mixtures thereof.

Such silylation reaction is performed at a suitable temperature which may vary according to the specific starting materials, and which is preferably in the range between room temperature (about 20°C) and 70 °C, more preferably in the range 40-60°C, even more preferably at 45-50°C.

The sylilation reaction is performed on a starting material or on an intermediate compound A bearing two functional groups, namely a carboxylic acid group and an amino group, and provides protection with a trimethylsilyl group to both functionalities, resulting in a bis-protected reactive compound, also referred to as a persilylated compound. Such compound is obtained *in situ* and it is then subjected to the subsequent reaction without being isolated from the reaction medium.

The persilylated compound then participates to a coupling reaction involving the trimethylsilyl-amino group which forms an amide bond with the optionally activated carboxylic group of a coupling partner compound B, which is another starting material or intermediate compound. The resulting coupling product is a compound wherein the carboxylic acid formerly protected as trimethylsilyl ester is a free carboxylic acid, according to the following scheme:

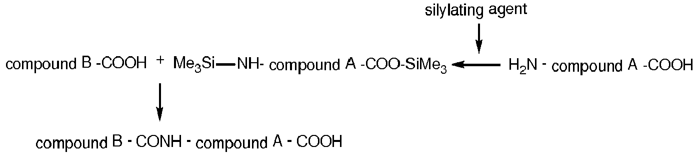

During the preparation of a compound of formula I according to the present invention, the carboxylic acid of the compound B involved in the amide bond formation is preferably activated prior to the coupling, either *in situ* or through an isolated compound.

In one embodiment of present invention, the activation of such carboxylic acid is carried out in the presence of a coupling reagent. The coupling reagent may be selected, among others, from the group comprising N,N'-diisopropylcarbodiimide (DIC), N,N'-dicyclohexylcarbodiimide (DCC), ethyl-dimethylaminopropyl carbodiimide (EDC), (Benzotriazol-1-yloxy)tripyrrolidinophosphonium hexafluorophosphate (PyBOP), N,N,N',N'-Tetramethyl-O-(benzotriazol-1-yl)uronium tetrafluoroborate (TBTU), 2-(7-Aza-1H-benzotriazole-1-yl)-1,1,3,3-tetramethyluronium hexafluorophosphate (HATU), 2-(1H-benzotriazole-1-yl)-1,1,3,3-tetramethyluronium hexafluorophosphate (HBTU), 2-(6-Chloro-1*H*-benzotriazole-1-yl)-1,1,3,3-tetramethylaminium hexafluorophosphate (HCTU), and the like. Preferably, the reaction is carried out in the presence of N,N'-diisopropylcarbodiimide or N,N'-dicyclohexylcarbodiimide.

In another embodiment of present invention, the activation of the carboxylic acid is performed in the presence of an additive or activating reagent. Additives form activated esters with carboxylic acids. The additive may be selected from the group comprising 1-hydroxybenzotriazole (HOBt), 2-hydroxypyridine N-oxide (HOPO), N-hydroxysuccinimide (HOSu or NHS), 1-hydroxy-7-azabenzotriazole (HOAt), endo-N-hydroxy-5-norbornene-2,3-dicarboxamide, ethyl 2-cyano-2-hydroxyimino-acetate (OxymaPure) and (1-cyano-2-ethoxy-2-oxoethylidenaminooxy)dimethylamino-morpholino-carbenium hexafluorophosphate (COMU).

Preferably, the activation reaction is carried out using N-hydroxysuccinimide with formation of an activated N-succinimidyl ester (OSu ester), which is more preferably isolated before further reaction.

Also preferably, the activation reaction is carried out using 2-hydroxypyridine N-oxide with formation of an activated N-hydroxy-2-pyridyl ester (OPO ester), which is generally not isolated but reacts *in situ* in the subsequent coupling reaction.

In a further preferred embodiment of present invention, the activation of the carboxylic acid is performed by its conversion to the acyl chloride by treatment with a chlorinating agent, like for instance thionyl chloride, phosphorus(V) chloride, and phosphorus(III) chloride. Preferably thionyl chloride is used. Generally the acyl chloride is not isolated but reacts *in situ* in the subsequent coupling reaction.

Preferred activating groups A are therefore OSu and OPO esters, or Cl (acyl chloride). The activation reaction is generally performed under standard conditions well known to the skilled person, for instance in a non protic organic solvent like for instance acetonitrile, tetrahydrofuran, dioxane, toluene, dichloromethane, ethyl acetate, isopropyl acetate and the like, or mixtures thereof.

Such activation reaction is performed at a suitable temperature which may vary according to the specific starting materials, and which is easily selected by the skilled person accordingly, and generally varies in the range between -20°C and 80°C, preferably between 0° and 50°C.

The coupling reaction is generally performed by mixing a solution of the activated compound A (also referred to as solution A) and a solution of the compound B (also referred to as solution B). In some embodiments, solution A is added to solution B; in some other embodiments solution B is added to solution A. The addition and the following reaction is performed at a suitable temperature, which may vary according to the specific starting materials, and which is easily selected by the skilled person accordingly.

With the term "C₁-C₆ alkyl", it is intended an aliphatic hydrocarbon chain, containing carbon-carbon single bonds only, which can be straight or branched. Representative examples include methyl, ethyl, n-propyl, isopropyl, n-butyl, isobutyl, tert-butyl, sec-butyl, n-pentyl, n-hexyl, and the like.

With the term "aryl", it is intended a mono-, bi- or poly-carbocyclic hydrocarbon with from 1 to 4 ring systems, optionally further fused or linked to each other by single bonds, wherein at least one of the carbocyclic rings is "aromatic", wherein the term "aromatic" refers to completely conjugated π-electron bond systems. Such aromatic rings may comprise from 1 to 3 heteroatoms, selected among N, O or S. Non limiting examples of such aryl groups are phenyl, biphenyl, pyridyl, pyrazinyl, pyrimidinyl, pyridazinyl, indolyl, imidazolyl, thiazolyl, isothiazolyl, pyrrolyl, furanyl, oxazolyl, isoxazolyl, pyrazolyl, thiophenyl groups, and the like.

With the term "arylalkyl", a combination of alkyl and aryl groups as defined above is intended.

When referring to a compound of formula I prepared by the process of the present invention, any stereoisomer is encompassed by its chemical structure. Any configuration is therefore encompassed at the alpha-carbon of the amino acids involved in the process of the invention. For this reason, configurations of stereogenic carbons are not indicated in the chemical structures depicted throughout the present disclosure.

As preferred embodiments, the process involves the use of amino acids like lysine and glutamic acid in their natural configuration L, even if lysine and glutamic acid of D configuration may be employed with comparable results.

The compound of formula I as prepared by the process of present invention is a useful building block in the synthesis of peptides, in particular in SPPS.

The compound of formula I is an alpha-amino protected amino acid substituted at its side-chain, that can be used, for instance, in the preparation of GLP-1 analogues, like liraglutide and semaglutide. In a preferred embodiment, a compound of formula I is a protected lysine substituted at its side-chain, that is used in position 20 of a GLP-1 analogue, like liraglutide or semaglutide.

The protective group PG to the alpha-amino position of a compound of formula I is selected from the groups that are stable in the reaction conditions wherein a group of formula VIII (i.e. Y, when it is not null) is involved, namely step a and step b (the conversion of a compound of formula Ia or Ib to a compound of formula I, which may comprise step d).

Such PG group is an amino protective group selected from the group comprising Fmoc, Boc, Alloc, benzoylcarbonyl (Cbz) and the like. Preferably, the PG group is selected from the group consisting of Fmoc, Boc, Alloc and Cbz. More preferably, PG is Fmoc group.

PG groups are generally cleaved in specific reaction conditions when they have completed their protective function. More PG groups can be used at the same time to protect different chemical functions on an intermediate compound, and can be cleaved at different stages of the synthesis, provided they require different chemical conditions, i.e. provided that they are orthogonal.

In a preferred embodiment, the process according to the present invention further comprises the preparation of the compound of formula X as defined above by using the same persilylation technique and according to the following steps:
step 1) reacting a persilylated compound of formula IV 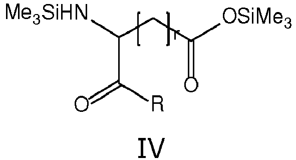 wherein r and R are as defined above, but, when R is OR2, R2 is not hydrogen, with a compound of formula III:

   R1-A III

   wherein R1 is as defined above and A is hydroxy or an activating group of a carboxylic acid;
step 2) optionally activating the resulting compound of formula Xa 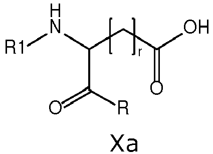 with an activating reagent as described above, such as to obtain a compound of formula X as defined above, wherein A is not hydroxy.

Preferred compounds of formula III are the following:

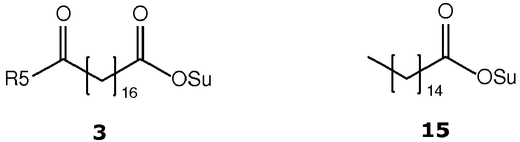

Preferred intermediates of formula X are the following: 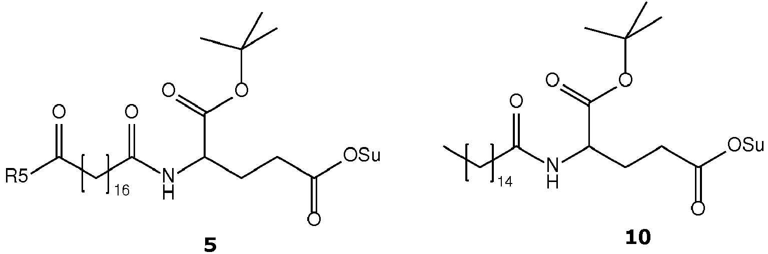 wherein an N-succinimidyl ester (OSu) is the activating group A.

Preferred persilylated intermediates of formula IXa, IXb, IXc and IXd, respectively, are the following:

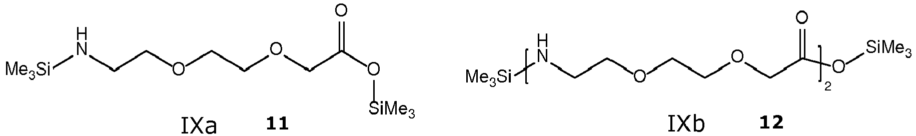

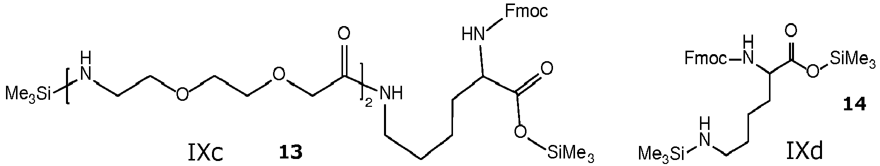

In another embodiment of present invention, a process for the preparation of a compound of formula I may involve reacting an intermediate compound of formula Xb 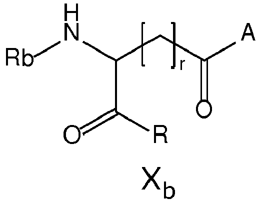 wherein Rb is R1 as defined above or PG1, which has the same meaning as PG, but is different from and orthogonal to PG.

The preparation of a compound of formula I in this latter specific case would involve a final coupling after deprotection of Rb, to be performed on a compound of formula Ix 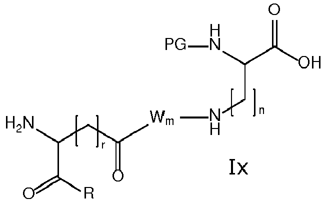 which could be persilylated and reacted with a compound of formula III R1-A as defined above, like for instance compound **3** or **15,** to finally provide a compound of formula I.

The convergent method for the preparation of protected amino acid derivatives of formula I according to the present invention provides a remarkable tool for the synthesis of peptides building blocks, which can greatly improve the overall yield and efficiency of the preparation of peptides analogues.

### Abbreviations

- SPPS: Solid Phase Peptide Synthesis
- LPPS: Liquid Phase Peptide Synthesis
- CSPPS: Convergent Solid Phase Peptide Synthesis
- CTC: 2-chloro-trityl chloride
- Fmoc: 9-Fluorenylmethoxycarbonyl
- Boc: *Tert*-butyloxycarbonyl
- Alloc: allyloxycarbonyl
- Trt: Trityl (triphenylmethyl)
- tBu: T*ert-*butyl
- Pbf: 2,2,4,6,7-Pentamethyl-dihydrobenzofuran-5-sulfonyl
- eq.: Equivalent
- h: hour/s
- min: minute/s
- RT: room temperature
- HPLC: High Performance Liquid Chromatography
- DIEA/DIPEA: N,N-Diisopropylethylamine
- DBU: 1,8-Diazabicyclo[5.4.0]undec-7-ene
- DMAP: 4-Dimethylaminopyridine
- TMA: N-methyl-N-trimethylsilylacetamide
- TFA: Trifluoroacetic acid
- TIS: Triisopropylsilane
- Ac₂O: Acetic anhydride
- ACN: Acetonitrile
- DMF: N,N-Dimethylformamide
- DMA: N,N-Dimethylacetamide
- DCM: Dichloromethane
- THF: Tetrahydrofuran
- NMP: N-Methyl-2-pyrrolidinone
- MTBE: Methyl-*tert*-butylether
- MeOH: Methanol
- HFIP: Hexafluoro-2-propanol
- TBDMS: Tert-butyl-dimethyl-silyl
- DIC: N,N'-Diisopropylcarbodiimide
- DCC: N,N'-Dicyclohexylcarbodiimide
- EDC: N-(3-dimethylaminopropyl)-N'-ethylcarbodiimide
- HOBt: 1-Hydroxybenzotriazole
- HOAt: 1-Hydroxy-7-azabenzotriazole
- EtOAc: Ethyl Acetate
- IprOAc: Isopropyl Acetate
- NHS: N-Hydroxysuccinimide
- HOPO: 2-hydroxypyridine N-oxide
- HOSu: N-Hydroxysuccinimide
- TBTU: O-(Benzotriazol-1-yl)-N, N,N', N'-tetramethyluronium tetrafluoroborate
- HBTU: O-(Benzotriazol-1-yl)-N,N,N',N'-tetramethyluronium hexafluorophosphate
- HATU: 2-(7-Aza-1H-benzotriazole-1-yl)-1,1,3,3-tetramethyluronium hexafluorophosphate
- HCTU: 2-(6-Chloro-1*H*-benzotriazole-1-yl)-1,1,3,3-tetramethylaminium hexafluorophosphate
- PyBOP: (Benzotriazol-1-yloxy)tripyrrolidinophosphonium hexafluorophosphate
- OxymaPure: Ethyl 2-cyano-2-hydroxyimino-acetate
- COMU: 1-Cyano-2-ethoxy-2-oxoethylidenaminooxy)dimethylamino-morpholino-carbenium hexafluorophosphate
- AEEA: 2-[2-(2-aminoethoxy)ethoxy]acetyl
- AEEA-OH: 2-[2-(2-Aminoethoxy)ethoxy]acetic acid

### Examples

Detailed experimental parameters suitable for the preparation of compounds of formula I according to the present invention are provided by the following examples, which are intended to be illustrative and not limiting of all possible embodiments of the invention.

Unless otherwise noted, all materials, solvents and reagents were obtained from commercial suppliers, of the best grade, and used without further purification.

### Example 1

### Preparation of Fmoc-Lys[(tBuOOC-(CH₂)₁₆-CO-γGlu-OtBu)-AEEA-AEEA]-OH (1)

Compound I or Ic (wherein m=2, n=4, r=2, R1=group XIII, t=16, R5=R=OR2, R2=tBu, W1=W2=group XI, p=q=1), through intermediate Ib

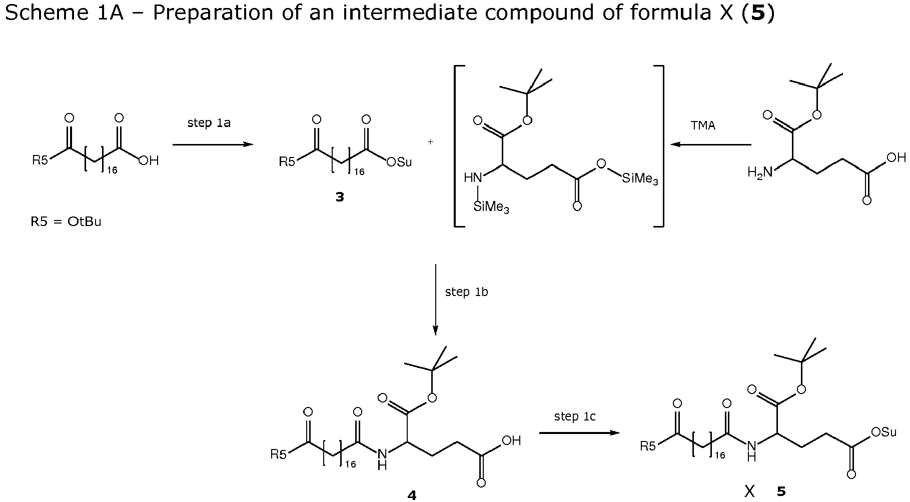

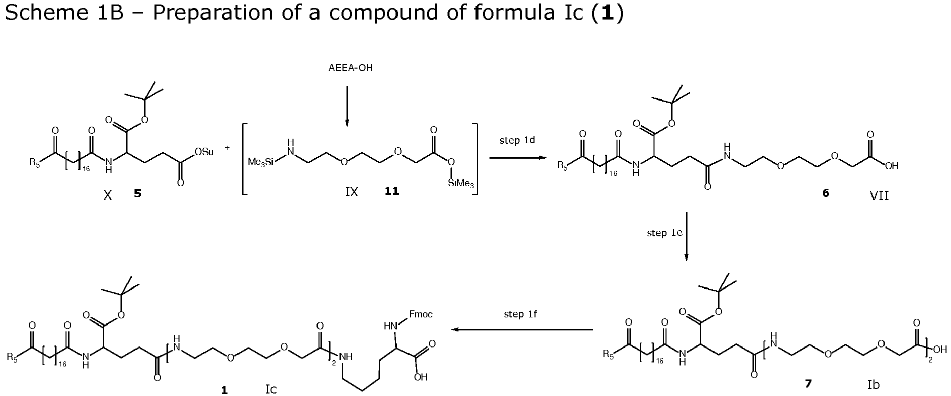

### Step 1a. Synthesis of tBuOOC-(CH₂)₁₆-COOSu (3, 18-tert-butoxv-18-oxooctadecanoic acid succinimidvl ester)

4 mmol (1.48 g) 18-tert-butoxy-18-oxooctadecanoic acid were dissolved at 40-45°C in 20 ml IprOAc in the presence of 4.4 mmol (0.51 g) HOSu. To the homogeneous solution 4.4 mmol (0.92 g) DCC were added in three portions over 15 min. The reaction was allowed to continue at this temperature for 3h, after which the suspension was filtered and the precipitate washed with 5 ml IprOAc. The solution was cooled to 0°C and kept stirring overnight. The suspension thus formed was filtered, washed with 2 ml of IprOAc and dried to achieve 1.85 g of the desired product (yield 92%).

### Step 1b. Synthesis of tBuOOC-(CH₂)₁₆-CO-Glu(OH)-OtBu (4)

0.69 g (3.75 mmol) of H-Glu(OH)-OtBu were suspended in 10 ml of ACN followed by the addition of 2.17 g (15 mmol) of TMA. The mixture was stirred until an homogeneous solution was obtained, then 1.4 g (3 mmol) of tBuOOC-(CH₂)₁₆-COOSu were added. The reaction was allowed to continue at room temperature overnight after which 300 microliters of water were added to the mixture. The mixture was thus stirred for 1h and then dried under vacuum. The residue was dissolved in 20 ml of DCM and extracted with 20 ml 5% KHSO₄. The organic phase was collected, the water phase extracted once more with 20 ml of DCM and the collected organic phases were dried under vacuum to small volume (2 ml). The mixture was left crystallizing at -20°C overnight to obtain 1.4 g of the desired product (yield 84%).

### Step 1c. Synthesis of tBuOOC-(CH₂)₁₆-CO-Glu(OSu)-OtBu (5)

The synthesis of tBuOOC-(CH₂)₁₆-CO-Glu(OSu)-OtBu ester was performed similarly to the synthesis described in step 1a.

4 mmol (2.16 g) tBuOOC-(CH₂)₁₆-CO-Glu(OH)-OtBu were dissolved at 40-45°C in 20 ml IprOAc in the presence of 4.4 mmol (0.51 g) HOSu. To the homogeneous solution 4.4 mmol (0.92 g) DCC were added in three portions over 15 min. The reaction was allowed to continue at this temperature for 3 h, after which the suspension was filtered and the precipitate washed with 5 ml IprOAc. The solution was cooled to 0°C and kept stirring overnight. The suspension thus formed was filtered, washed with 2 ml of IprOAc and dried to achieve 2.27 g of the desired product (yield 89%).

### Step 1d. Coupling of tBuOOC-(CH₂)₁₆-CO-Glu(OSu)-OtBu to AEEA acid via persilylation method

2-[2-(2-Aminoethoxy)ethoxy]acetic acid (AEEA-OH, 897 mg, 5.5 mmol) was suspended in 10 ml ACN followed by the addition of 22 mmol TMA (3.2 g). The suspension was warmed to 45-50°C till a homogeneous solution was obtained. The solution was cooled to room temperature and diluted with 20 ml ACN, whereafter 5 mmol tBuOOC-(CH₂)₁₆-CO-Glu(OSu)-OtBu (3.19 g) were added. The mixture was stirred until reaction completion after which 1 ml water was added. The solution was taken to concentrated under vacuum to dryness and the residue partitioned between 60 ml 10% KHSO₄ and 60 ml DCM. The organic phase was recovered and washed twice with brine solution. The DCM phase was concentrated to dryness and the residue was suspended with 60 ml diisopropylether. The mixture was stirred at room temperature for 30 min, filtered, the cake washed twice with diisopropylether (10 ml each wash) to obtain 2.81 g of tBuOOC-(CH₂)₁₆-CO-Glu(AEEA)-OtBu **(6,** yield 82%).

### Step 1e. Coupling of tBuOOC-(CH₂)₁₆-CO-Glu(AEEA-OH)-OtBu (6) to AEEA-OH via persilylation method

Solution A: 5.5 mmol AEEA-OH (897 mg) were suspended in 10 ml ACN followed by the addition of 22 mmol TMA (3.2 g). The suspension was warmed to 45-50°C till a homogeneous solution was obtained. The solution comprising persilylated AEEA (11) was cooled down to room temperature.

Solution B: 5 mmol tBuOOC-(CH₂)₁₆-CO-Glu(AEEA-OH)-OtBu (3.43 g) and 5.5 mmol HOPO (610 mg) were dissolved in 50 ml ACN whereafter 5.5 mmol DCC (1.13 g) were added. The mixture was stirred for 2h at room temperature.

Coupling: Solution A was added to solution B. The mixture was stirred at room temperature until reaction completion and 1 ml of water was added. The mixture was filtered, dried under vacuum to solid residue and partitioned between 60 ml 10% KHSO₄ and 60 ml DCM. The organic phase was recovered and washed twice with brine solution. The DCM phase was concentrated to dryness and the residue was suspended with 60 ml diisopropylether. The mixture was stirred at room temperature for 30 min, filtered, the cake washed twice with diisopropylether (10 ml each wash) to obtain 3.28 g of product **(7,** yield 79%).

### Step 1f. Coupling of tBuOOC-(CH₂)₁₆-CO-Glu(AEEA-AEEA)-OtBu (7) to persilylated Fmoc-Lvs

Solution A: 5.5 mmol Fmoc-Lys-OH (2.03 g) were suspended in 20 ml DCM followed by the addition of 22 mmol TMA (3.2 g). The suspension was warmed to 45-50°C till a homogeneous solution was obtained. The solution comprising persilylated Fmoc-Lys (14) was cooled down to room temperature.

Solution B: 5 mmol tBuOOC-(CH₂)₁₆-CO-Glu(AEEA-AEEA)-OtBu **(7,** 4.16 g) obtained as described in step 1e and 5.5 mmol HOPO (610 mg) were dissolved in 50 ml DCM whereafter 5.5 mmol DCC (1.13 g) were added. The mixture was stirred for 2h at room temperature.

Coupling: Solution A was added to solution B. The mixture was stirred at room temperature until reaction completion, then 1 ml water was added. The mixture was filtered, dried under vacuum to solid residue and partitioned between 60 ml 10% KHSO₄ and 60 ml DCM. The organic phase was recovered and washed twice with brine solution. The DCM phase was concentrated to dryness and the residue was suspended with 60 ml diisopropylether. The mixture was stirred at room temperature for 30 min, filtered, the cake washed twice with diisopropylether (10 ml each wash) to obtain 4.43 g of final product **(1,** yield 75%).

### Example 2

### Preparation of Fmoc-Lys[(tBuOOC-(CH₂)₁₆-CO-γGlu-OtBu)-AEEA-AEEA]-OH (1)

Compound I or Ic (wherein m=2, n=4, r=2, R1=group XIII, t=16, R5=R=OR2, R2=tBu, W1=W2=group XI, p=q=1), through intermediate Ib

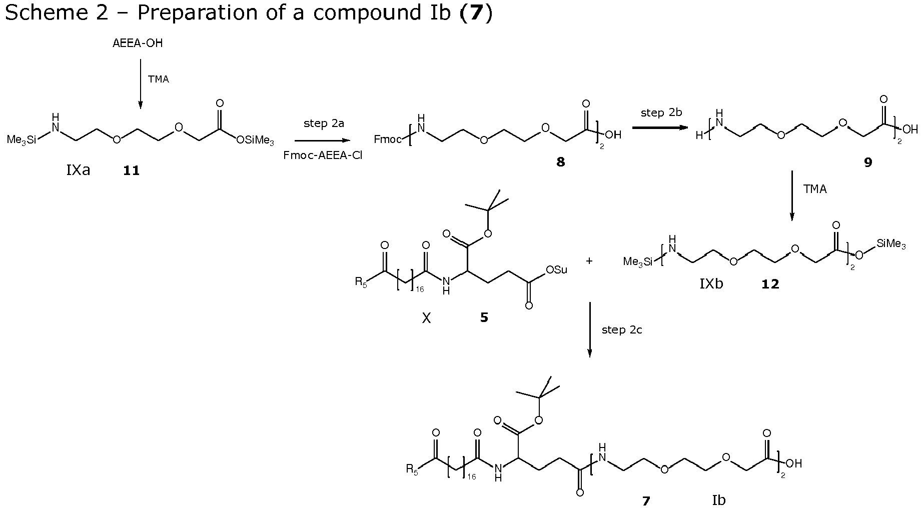

### Step 2a. Coupling of Fmoc-AEEA-OH to AEEA-OH via persilylation method

Solution A: 6 mmol AEEA-OH (978 mg) were suspended in 20 ml DCM followed by the addition of 22 mmol TMA (3.2 g). The suspension was warmed to 45-50°C till a homogeneous solution was obtained. The solution was cooled down to -10°C.

Solution B: 5.5 mmol Fmoc-AEEA-OH (2.12 g) were converted to its acyl chloride by dissolving in 10 ml DCM followed by the addition of 3.5 ml thionylchloride. After 1h the solution was concentrated, dissolved in 20 ml toluene and again concentrated. The residue was dissolved in 10 ml DCM.

Coupling: solution B was slowly added to the solution A. The mixture was stirred for 2h, then 1 ml of water was added. The solution was successively treated with 30 ml 10% KHSO₄ and 2 x 30 ml of brine. The organic phase was taken to dryness and used as such in the following deprotection step.

### Step 2b. Cleavage of the Fmoc group

The residue obtained in step 2a was taken up in 25 ml ethanol and treated with 50 mmol (3.65 g) diethylamine. After completion of the reaction the solution was taken to dryness. The residue was partitioned between 25 ml DCM and 25 ml water. The water phase was lyophilized to obtain 1.7 g of the desired product AEEA-AEEA-OH **(9,** yield 90%).

### Step 2c. Coupling of tBuOOC-(CH₂)₁₆-CO-Glu(OSu)-OtBu (5) to persilvlated AEEA-AEEA-OH

5.5 mmol AEEA-AEEA-OH **(9,** 1.7 g) were suspended in 20 ml ACN followed by the addition of 22 mmol TMA (3.2 g). The suspension was warmed to 45-50°C till a homogeneous solution was obtained. The solution was further diluted with 20 ml ACN and 5 mmol of tBuOOC-(CH₂)₁₆-CO-Glu(OSu)-OtBu ester **(5,** 3.19 g) prepared as described in step 1c were added. The mixture was kept stirring at room temperature until reaction completion, then 1ml of water was added. The solution was taken to dryness by evaporation and the residue partitioned between 60 ml 10% KHSO₄ and 60 ml DCM. The organic phase was recovered and washed twice more with brine solution. The DCM phase was concentrated to dryness and the residue was diluted with 60 ml diisopropylether. The mixture was stirred at room temperature for 30 min, filtered, the cake washed twice with diisopropylether (15 ml each wash) to obtain 3.12 g of tBuOOC-(CH₂)₁₆-CO-Glu(AEEA-AEEA-OH)-OtBu (**7**, yield 75%).

The preparation of the final product **1** was then completed as described in Example 1, step 1f.

### Example 3

### Preparation of Fmoc-Lys(N-(1-oxohexadecy))-L-γ-glutamyl-OtBu)-OH (2) (i.e. Fmoc-Lys(Pal-Glu-OtBu)-OH)

Compound I or Id (wherein m=0, n=4, r=2, R=OR2=OtBu, R1=group XII, s=14)

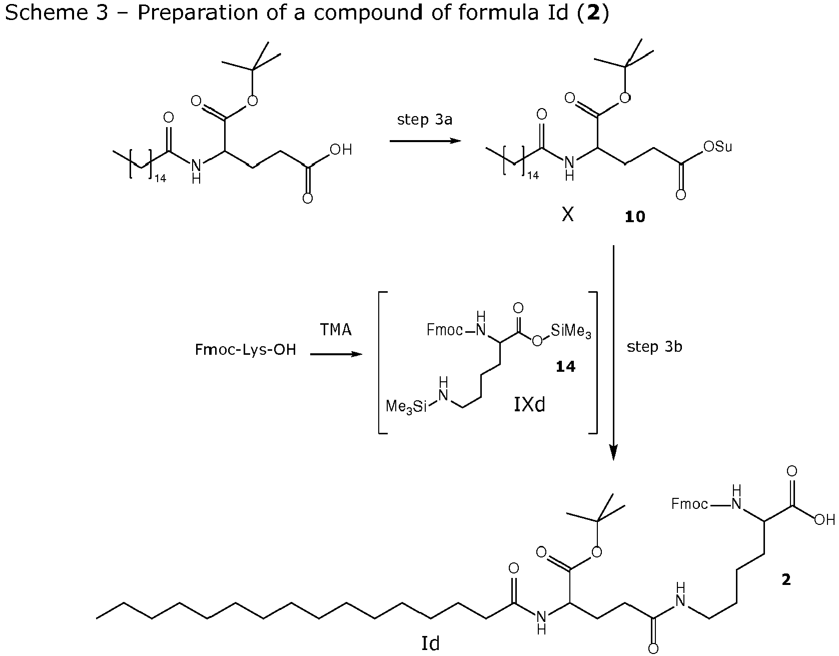

### Step 3a: Synthesis of Pal-Glu(OSu)-OtBu ester (10)

23.6 g (53.5 mmol) of Pal-Glu-OtBu and 6.7 g (58 mmol) HOSu were dissolved in a mixture of 300 ml EtOAc and 125 ml ACN at RT. To this mixture, a solution of 13.9 g DCC (58 mmol) in 75 ml EtOAc was added, over 20 min. After 6 h the reaction mixture was filtered and the solid washed with 2x50 ml (EtOAc: ACN 3:1). The filtrate and the washing were concentrated and the residue taken up in 150 ml warm isopropanol. The solution was left to crystallize overnight at 4°C. The suspension thus obtained was filtered, washed with 50 ml Et₂O and dried to achieve 22.1 g of the desired product **(10,** yield 82%).

### Step 3b: Coupling of Pal-Glu(Osu)-OtBu ester (10) to persilvlated Fmoc-Lvs-OH

16.6 g (45 mmol) Fmoc-Lys-OH were suspended in 675 ml DCM and warmed to 40°C. 20.25 g (140 mmol) TMA were added. The mixture was stirred until a clear solution was observed and 23.4 g Pal-Glu(OSu)-OtBu were added as a solid. The mixture was left stirring for 2 h at 40°C whereafter it was washed twice with 340 ml 2.5% KHSO₄ (each wash) and lastly with 340 ml water. The organic layer was recovered and concentrated up to solid residue. The residue was re-dissolved in 500 ml ethanol and re-concentrated to 50 ml. The residue was left overnight at room temperature during which the formation of crystals occurred. The suspension was filtered, washed with a mixture of EtOH:H₂O 2:3 (50 ml) and dried to obtain 28 g of the final product **(2,** yield 78%, HPLC purity 99.6%).

## Claims

1. A process for the preparation of a compound of formula I or a salt thereof, 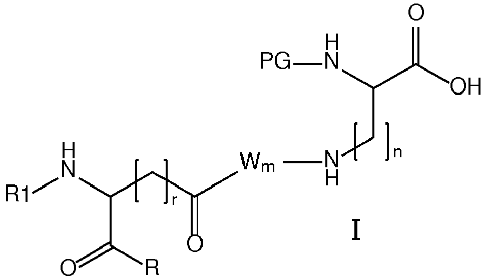 wherein
PG is an amino protective group selected from the group consisting of Fmoc, Boc, Alloc, benzoylcarbonyl and their derivatives,
n is an integer from 1 to 10;
m is 0 or 2;
r is an integer from 0 to 10;
each W is independently a group of formula XI 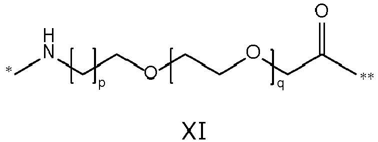 wherein
p is an integer from 1 to 3;
q is an integer from 1 to 2;
* denotes the point of attachment to the carbonyl in formula I or to the C-terminal end of the further group W;
** denotes the point of attachment to NH in formula I or to the N-terminal end of the further group W;
R is OR2 or NR3R4, wherein R2, R3 and R4 are selected from the group consisting of hydrogen, linear or branched C₁-C₆ alkyl, aryl and arylalkyl;
R1 is selected from a group of formula XII and a group of formula XIII: 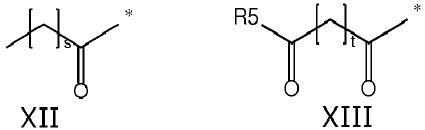 wherein
* denotes the point of attachment to NH;
R5 is OR2 or NR3R4, as defined above;
s is an integer from 0 to 20
t is an integer from 1 to 20;
such process comprising:
step a) reacting a compound of formula X 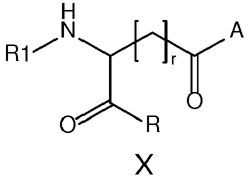 wherein A is OH or an activating group, with a persilylated compound of formula IX 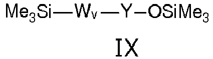 wherein
v is 0, 1 or 2, and Y is null or a group of formula VIII 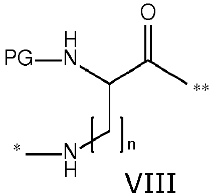 wherein
* denotes the point of attachment to Wᵥ or to SiMe₃;
** denotes the point of attachment to OSiMe₃, and
n is as defined above;
and which is selected from 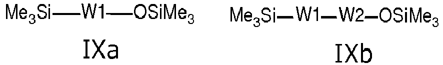 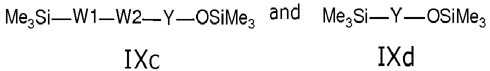
wherein Y is not null and wherein W1 and W2 are the same or different W groups, as defined above;
such reaction resulting in a compound of formula Iv: 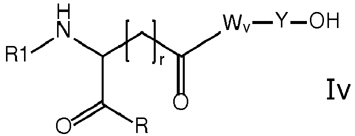 which is a compound of formula: 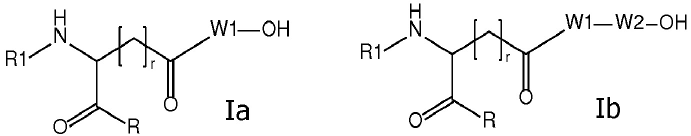 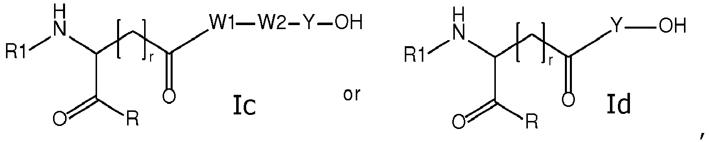 respectively;
step b) optionally converting a compound of formula Ia or Ib to a compound of formula I.

2. The process according to claim 1, wherein
step c) a compound of formula Ia, optionally activated at its carboxylic acid with a group A, is reacted with a compound of formula IXe 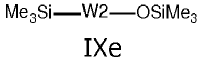 resulting in a compound of formula Ib.

3. The process according to claim 1 or claim 2, wherein
step d) a compound of formula Ib, optionally activated at its carboxylic acid with a group A, is reacted with a compound of formula IXd, resulting in a compound of formula Ic.

4. The process according to any of the preceding claims, further comprising:
step 1) reacting a persilylated compound of formula IV 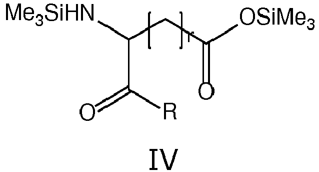 with a compound of formula III
R1-A III
wherein R, R1 and r are as defined in claim 1 and A is hydroxy or an activating group of a carboxylic acid;
step 2) optionally activating the resulting compound of formula Xa 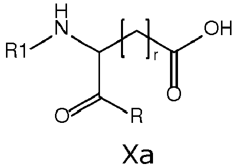 with an activating reagent, such as to obtain a compound of formula X, wherein A is not hydroxy.

5. The process according to any of the preceding claims, for the preparation of a compound of formula I wherein n is 4 and r is 2.

6. The process according to claim 5, for the preparation of a compound of formula I wherein m is 0, R is OR2 wherein R2 is tert-butyl, and R1 is 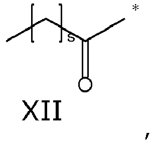 wherein s is 14.

7. The process according to claim 5, for the preparation of a compound of formula I wherein m is 2, R is OR2, wherein R2 is tert-butyl, and R1 is 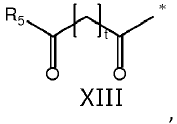 wherein t is 16 and R5 is OR2, wherein R2 is tert-butyl, and
wherein W1 and W2 are the same group of formula XI as defined in claim 1, wherein p and q are 1.

8. The process according to any of the preceding claims, wherein the persilylated compounds are obtained by using a silylating reagent, selected in the group consisting of N-methyl-N-trimethylsilylacetamide, trimethylsilyl cyanide, bis(trimethylsilyl)acetamide, 1,3-bis(trimethylsilyl)urea, trimethylchlorosilane, and trimethyliodosilane.

9. The process according to claim 7, wherein the persilylated compounds are obtained *in situ* by using N-methyl-N-trimethylsilylacetamide.

10. The process according to any of claims 1, 2, 3 or 4, wherein the activating group A of a compound of formula X, Ia, Ib or III is an activated ester or an acyl chloride.

11. The process according to claim 10, wherein the activating group A is an activated ester, obtainable by using an additive selected in the group consisting of 1-hydroxybenzotriazole, 2-hydroxypyridine N-oxide, N-hydroxysuccinimide, 1-hydroxy-7-azabenzotriazole, endo-N-hydroxy-5-norbornene-2,3-dicarboxamide, ethyl 2-cyano-2-hydroxyimino-acetate and (1-Cyano-2-ethoxy-2-oxoethylidenaminooxy)dimethylamino-morpholino-carbenium hexafluorophosphate.

12. The process according to claim 6, for the preparation of a compound of formula I which is compound **2:** 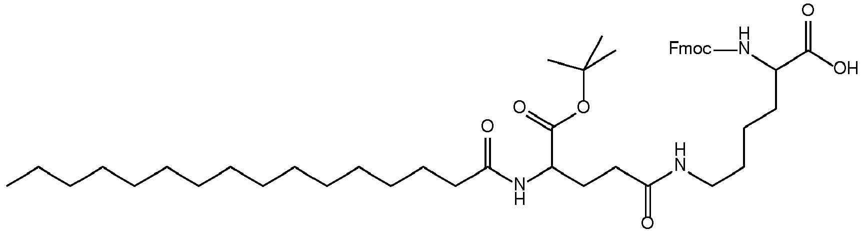 wherein configuration at both stereocenters is L.

13. Use of the compound of formula I according to claim 12 and prepared according to the process of claim 1 for the synthesis of liraglutide.

14. The process according to claim 7, for the preparation of a compound of formula I which is compound **1:** 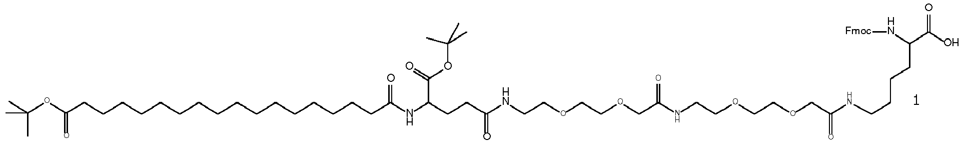 wherein configuration at both stereocenters is L.

15. Use of the compound of formula I according to claim 14 and prepared according to the process of claim 1 for the synthesis of semaglutide.
